Europäisches Patentamt

European Patent Office

Office européen des brevets

(19)

(11) **EP 1 498 134 A1**

(12) **EUROPEAN PATENT APPLICATION**
published in accordance with Art. 158(3) EPC

(43) Date of publication:
**19.01.2005 Bulletin 2005/03**

(21) Application number: **03701878.5**

(22) Date of filing: **27.01.2003**

(51) Int Cl.⁷: **A61K 38/00**, A61K 45/00,
A61K 35/20, A61K 31/7004,
A61K 31/7016, A61P 1/04,
A61K 31/04, A23C 9/156,
A23F 3/00, A23F 5/00,
A23L 1/22, A23L 1/40,
A23L 2/00

(86) International application number:
**PCT/JP2003/000724**

(87) International publication number:
**WO 2003/063886 (07.08.2003 Gazette 2003/32)**

(84) Designated Contracting States:
**AT BE BG CH CY CZ DE DK EE ES FI FR GB GR
HU IE IT LI LU MC NL PT SE SI SK TR**
Designated Extension States:
**AL LT LV MK RO**

(30) Priority: **28.01.2002 JP 2002018606**

(71) Applicants:
• **Nisshin Pharma Inc.
Tokyo 101-8441 (JP)**
• **Ghen Corporation
Gifu-shi Gifu-ken 501-1132 (JP)**

(72) Inventors:
• **HIRAMOTO, Shigeru c/o Nisshin Pharma Inc.
Iruma-gun, saitama 356-8511 (JP)**

• **MORISHITA, Yoshiro
c/o Nisshin Kyorin Pharma.Co, L
Iruma-gun, Saitama 356-8511 (JP)**
• **KIMURA, Nobutake c/o Nisshin Pharma Inc.
Iruma-gun, Saitama 356-851 1 (JP)**
• **KODAMA, Yoshikatsu
Ghen Corporation, Immunology
Gifu-shi, Gifu 501-1101 (JP)**

(74) Representative: **Schrimpf, Robert et al
Cabinet Regimbeau
20, rue de Chazelles
75847 Paris cedex 17 (FR)**

(54) **HELICOBACTER PYLORI ADHESION INHIBITOR**

(57)    This invention relates to a *Helicobacter pylori* adhesion inhibitor that is capable of eliminating *Helicobacter pylori* associated with the development of peptic ulcer from the stomach, a method for producing the same, and pharmaceutical compositions and foods for preventing or treating diseases associated with *Helicobacter pylori* comprising such *Helicobacter pylori* adhesion inhibitor. The inhibitor of the present invention has excellent activity of eradicating *Helicobacter pylori* and is highly safe. Accordingly, pharmaceutical compositions and foods comprising the same are highly useful for the prevention or treatment of the aforementioned diseases.

**Description**

BACKGROUND OF THE INVENTION

Field of the Invention

The present invention relates to a *Helicobacter pylori* adhesion inhibitor that is capable of eliminating *Helicobacter pylori* associated with the development of peptic ulcer from the stomach, a method for producing the same, and pharmaceutical compositions and foods for preventing or treating digestive diseases associated with *Helicobacter pylori* that comprise this inhibitor.

Prior Art

*Helicobacter pylori* is a type of spiral gram-negative bacillus, which has several flagellea at its one end and inhabits the human gastric mucosa. In 1983, Marshall, B. J. and Warren, J. R. from Australia reported that this bacterium was detectable with high efficiency from gastric biopsy specimens sampled from patients with gastritis or gastric ulcer. Since this time, many reports have been successively made concerning this bacterium. As a result of epidemiologic study, for example, this bacterium is reported to cause gastritis, gastric ulcer, and duodenal ulcer, and to be associated with diseases such as gastric cancer.

At present, accordingly, eradication of *Helicobacter pylori* is considered to be essential for a permanent cure for peptic ulcer. Combination therapy employing antibiotics and inhibitors of gastric-acid secretion is widely used as an eradication treatment as described below.

Once *Helicobacter pylori* colonizes the gastric mucosa, it continuously inhabits the stomach without being eradicated in spite of potent immune response to infection (i.e., high antibody titer). Thus, the condition of the patient returns to that before treatment (i.e., the infected state) within about a month after the discontinuation of drug administration, unless *Helicobacter pylori* is completely eradicated by means of antibiotic therapy. Since the pH level in the stomach is kept very low by hydrochloric acid, many antibiotics are inactivated. Thus, proton pump inhibitors that potently inhibit gastric-acid secretion are used in combination with a bacteria-eliminating agent (antibiotic) to eradicate *Helicobacter pylori*. At present, combination therapy utilizing three types of agents, such as amoxicillin, clarithromycin, and lansoprazole, is a standard therapy for *Helicobacler pylori* eradication in Japan. The long-term administration of antibiotics, however, could cause very serious problems, such as an increased number of drug-resistant strains as well as side effects.

At present, an immunotherapeutic approach using oral vaccines is available as a means for solving the problems of side effects or an increased number of drug-resistant strains caused by the administration of antibiotics for the eradication of bacteria. However, research aiming at establishing a novel preventive or therapeutic method has made substantially no progress due to the complex conditions of experimentation. Also, the principal purpose of the vaccine administration is prevention. Therefore, it cannot be effective for patients who have already been infected with *Helicobacter pylori.*

In general, bacteria first need to adhere to, propagate in, and colonize host cells to establish bacterial infection. The adhesion of bacteria to host cells requires binding of adhesins to receptors on the host cell surface. JP 10-287585 A1 (1998) discloses the mechanism of adhesion of *Helicobacter pylori.* That is, the adhesins, which have not been elucidated, are ureases produced from the bacteria, and an antibody from chicken egg against such ureases significantly suppresses the proliferation of *Helicobacter pylori* in the stomach.

JP 3,255,161 discloses that mucin derived from whey obtained by removing milk fat and casein from milk can inhibit colonization of *Helicobacter pylori* in the alimentary canal.

However, it is still unknown whether or not a product of browning reaction of a variety of sugar and protein can inhibit the adhesion of a *Helicobacter pylori* adhesin, urease, to the gastric mucosa.

SUMMARY OF THE INVENTION

An object of the present invention is to provide a highly safe *Helicobacter pylori* adhesion inhibitor that can effectively inhibit the adhesion of *Helicobacter pylori* associated with the development of peptic ulcer to the gastric mucosa, where such adhesion inhibitor does not involve problems such as side effects or an increased number of drug-resistant strains caused by the use of conventional antibiotics.

The present inventors have examined a variety of substances that can inhibit urease from adhering to the gastric mucosa. As a result, they have found that the product of browning reaction of sugar and protein is capable of effectively inhibiting urease, as an adhesin, from adhering to the gastric mucosa. This has led to the completion of the present invention.

More specifically, the present invention relates to a *Helicobacter pylori* adhesion inhibitor comprising, as an active ingredient, a product of browning reaction of sugar and protein. Any orally-ingestible proteins and any orally-ingestible reducing sugars may be employed in the present invention.

The present invention also relates to a method for producing a *Helicobacter pylori* adhesion inhibitor comprising a step of subjecting sugar and protein to a browning reaction in an aqueous solution.

Further, the present invention relates to a method for producing a *Helicobacter pylori* adhesion inhibitor comprising a step of subjecting food containing sugar and protein to a browning reaction in an aqueous solution. In this invention, the browning reaction is carried out until absorbance at 405 nm becomes at least 0.01 in a 5% aqueous solution of a sugar-protein mixture. Foods are not particularly limited as long as they contain sugars and proteins. Preferable examples thereof include raw cow's milk, milk powder (whole milk powder), skim milk powder, whey, and evaporated milk (condensed milk).

The present invention relates to a *Helicobacter pylori* adhesion inhibitor comprising a product of browning reaction of sugar and protein, and an inhibitor of gastric-acid secretion. The invention also relates to a *Helicobacter pylori* adhesion inhibitor comprising a product of browning reaction of sugar and protein, and other substances capable of eradicating *Helicobacter pylori*. Further, the present invention relates to a *Helicobacter pylori* adhesion inhibitor comprising a product of browning reaction of sugar and protein, an inhibitor of gastric-acid secretion, and other substances capable of eradicating *Helicobacter pylori.* In the invention, the term "capable of eradicating" refers to actions such as adhesion inhibition, suppression or prevention of proliferation, and sterilization of *Helicobacter pylori in vivo* and/or in *vitro*.

The invention also relates to a pharlaceutical composition for preventing or treating diseases associated with *Helicobacter pylori*, which comprises the aforementioned *Helicobacter pylori* adhesion inhibitor. Further, the invention relates to food for preventing or ameliorating diseases associated with *Helicobacter pylori,* which comprises the aforementioned *Helicobacter pylori* adhesion inhibitor.

DETAILED DESCRIPTION OF THE INVENTION

Hereinafter, the present invention will be described in detail.

An active ingredient of the *Helicobacter pylori* adhesion inhibitor of the present invention is a product of browning reaction of sugar and protein, that is, a product of an amino-carbonyl reaction. The browning reaction can be carried out by mixing a variety of sugars and a variety of proteins and heating the mixtures in an aqueous solution.

In the invention, any orally-ingestible proteins can be used. Examples thereof include, but are not limited to, plant proteins derived from wheat, barley, rice, corn, soybeans, red beans, or the like, and animal proteins derived from milk, eggs, fish, meat, or the like. Specific examples thereof include: casein, β-lactoglobulin, α-lactalbumin, bovine serum albumin, immunoglobulin, or lactoferrin contained in milk; glutenin or albumin contained in wheat; zein contained in corn; ovalbumin, ovotransferrin, ovomucoid, ovomucin, or lysozyme contained in eggs; and myosin or actin contained in fish or meat. These proteins may be purified in accordance with conventional techniques before use, or commercialized proteins may be used without processing. These proteins may be used solely or in combinations of two or more. Alternatively, food materials containing such proteins, for example, raw cow's milk, milk powder, skim milk powder, whey, or evaporated milk containing milk-derived protein, may be used as protein mixtures.

Examples of sugars for producing a product of browning reaction include a variety of reducing sugars such as D-glucose, D-fructose, D-mannose, D-galactose, D-xylose, L-arabinose, D-ribose, and lactose. Purified products of these sugars may be used. When skim milk powder or whey is used, however, the browning reaction may be carried out without further addition of milk-derived proteins since these substances already contain proteins as mentioned above.

The mass ratio of sugars to proteins may be at any level. In general, it is between 1:100 and 10:1, and preferably between 1:9 and 1:1.

In the present invention, a product of browning reaction can be prepared by mixing sugar and protein in an aqueous solution as mentioned above. Alternatively, food containing sugar and protein, preferably food containing sugar and protein in amounts mentioned above, such as raw cow's milk, milk powder, skim milk powder, whey, or evaporated milk, can be heated in an aqueous solution in that state, and a browning reaction is allowed to occur to give a product of browning reaction.

Skim milk powder contains a variety of milk proteins, mostly casein, and sugar such as lactose. Skim milk powder can be obtained by centrifuging raw milk by conventional techniques to separate and remove fat and pulverizing the resulting skim milk. Alternatively, commercialized skim milk powder may also be used.

Whey is prepared by removing casein from skim milk, and it contains sugar such as lactose and whey protein such as β-lactoglobulin, α-lactalbumin, serum albumin, immunoglobulin, or lactoferrin. In the invention, whey may be prepared from a cow's milk in accordance with a conventional technique, or commercialized whey (e.g., a condensate or powder) may be used without processing.

In the present invention, the browning reaction is preferably carried out in a neutral or alkaline aqueous solution. A variety of alkaline aqueous solutions can be used, and examples thereof are alkaline aqueous solutions of sodium hydroxide, potassium hydroxide, sodium bicarbonate (baking soda), sodium carbonate, and disodium phosphate. The concentration of alkali to be added is not particularly limited. For example, 0.05N to 0.5N sodium hydroxide can be used. The amount of an aqueous solution is not particularly limited as long as the solution can homogenously suspend a sugar-protein mixture or food containing sugar and protein. For example, the volume of the solution may be 1 to 100 times, and preferably 5 to 20 times, the volume of a mixture or food.

The browning reaction is carried out until absorbance at 405 nm becomes at least 0.01, and preferably at least 0.1, in a 5% aqueous solution of a sugar-protein mixture. Absorbance of at least 0.01 at 405 nm means that the browning reaction is in progress. Absorbance is determined by, for example, assaying 100 μl of a 5% aqueous solution using a microplate reader.

When the browning reaction is carried out in a neutral aqueous solution, the reaction temperature is preferably at least 100°C, and particularly preferably at least 120°C. The reaction period is generally at least 20 minutes, and preferably between 30 minutes and 10 hours. When the browning reaction is carried out in an alkaline aqueous solution, the reaction temperature is preferably between room temperature and 100°C, particularly preferably between 40°C and 90°C. The reaction period is generally between 1 hour and 20 hours, preferably between 2 hours and 8 hours.

The product of browning reaction may be ingested without further processing. Alternatively, it may be subjected to common techniques, for example, ultrafiltration or ion-exchange resins, for removal of unreacted sugar and desalting. The product of browning reaction may be precipitated by conventional techniques that are employed for selective precipitation of proteins, such as isoelectric precipitation, salting-out, or organic solvent precipitation. The product of browning reaction is preferably dehydrated by common techniques, such as lyophilization or spray drying, in order to facilitate the preparation thereof into pharmaceutical compositions or the addition thereof to foods. When the browning reaction is carried out in a neutral aqueous solution, the product may be dehydrated without further processing. Preferably, the product is pulverized by removing unreacted sugar and desalting, followed by dehydration by the aforementioned techniques. When the browning reaction is carried out in an alkaline aqueous solution, the solution may be neutralized with acid (inorganic and/or organic acid). After the neutralization, the solution may be dehydrated without further processing. Alternatively, unreacted sugar may be removed from the solution, and the solution may be desalted by the aforementioned technique, followed by dehydration. The product can be then pulverized.

The product of browning reaction of the present invention preferentially binds to a *Helicobacter pylori* adhesin, urease, thereby inhibiting the adhesion thereof to the receptor on the gastric mucosa. The product of browning reaction of the invention has the excellent capacity for eradicating *Helicobacter pylori*, and thus is useful for prevention or amelioration of diseases associated with *Helicobacter pylori,* such as peptic ulcer. Also, starting materials such as sugar and protein are foods or orally-ingestible substances derived from food or food materials, and browning reactions constantly occur in the process of cooking foods. Accordingly, the product of browning reaction of the invention is highly safe and does not cause side effects. If the *Helicobacter pylori* adhesion inhibitor containing the product of browning reaction of the invention is incorporated in pharmaceutical compositions or foods, they can prevent or ameliorate diseases associated with *Helicobacter pylori*.

An adhesion inhibitor comprising the product of browning reaction of the present invention can be formulated by subjecting the product alone or in suitable combination with a carrier or excipient (e.g., a filler or binder) according to the conventional techniques for preparing pharmaceuticals. Other additives or agents, for example, antacids (e.g., sodium bicarbonate, magnesium carbonate, precipitated calcium carbonate, or hydrotalcite), gastric mucosa protectants (e.g., synthetic aluminum silicate, sucralfate, or sodium copper chlorophyllin), or digestive enzymes (e.g., biodiastase or lipase) may be added according to need. The adhesion inhibitor of the invention is administered orally, and the dose is generally in the range of 0.01 g to 10.0 g (on a dry basis), preferably in the range of 0.5 g to 5.0 g, of the product of browning reaction per day per adult.

The effects of the adhesion inhibitor comprising the product of browning reaction of the invention can be enhanced through combined use with an inhibitor of gastric-acid secretion. Examples of an inhibitor of gastric-acid secretion that can be used include, but are not limited to, $H_2$-blockers, such as famotidine, nizatidine, roxatidine, ranitidine, and cimetidine, and proton pump inhibitors, such as omeprazole, lansoprazole, and rabeprazole sodium. Acid reducer dosages vary depending on the type of agent used. Dosages are generally in the range of 10 mg to 50 mg, and preferably in the range of 20 mg to 30 mg, per day per adult.

The effect of eradicating *Helicobacter pylori* of the adhesion inhibitor of the present invention can be further enhanced when it is used in combination with other substances capable of eradicating *Helicobacter pylori.* Examples of such other substances include a variety of antibiotics, polyphenol, an antibody against *Helicobacter pylori,* and polysaccharide or glycoprotein capable of binding to a *Helicobacter pylori* adhesin, urease. Further, the adhesion inhibitor of the invention may be combined with a therapeutic agent for gastric diseases, such as a bismuth preparation, or a parasiticide, such as metronidazole.

Examples of polyphenol include catechin, gallocatechin, gallocatechin gallate, epicatechin, epicatechin gallate, epigallocatechin, epigallocatechin gallate, free theaflavin, theaflavin monogallate, theaflavin gallate, resveratrol, proanthocyanidin, quercetin, anthocyanin, sulforaphane, and isoflavone. The adhesion inhibitor of the present invention may also contain such polyphenol. The adhesion inhibitor of the invention may be incorporated in tea containing such polyphenol, food materials such as cacao or fruit, or extracts thereof.

Examples of an antibody against *Helicobacter pylori* include an antibody against the entire cell and an antibody against a cell-surface molecule. From the viewpoints of availability and enhanced eradication, an antibody from chicken egg against a cell-surface molecule, and particularly a antibody from chicken egg against urease associated with *Helicobacter pylori* adhesion and flagella (JP 10-28758 A (1998)), is preferable.

Examples of glycoproteins capable of binding to *Helicobacter pylori* urease include mucin derived from the mammalian alimentary canal, mucin derived from whey, mucin derived from chicken egg albumen, and glycoproteins constituting these mucins. Examples of polysaccharides capable of binding to the aforementioned urease include dextran sulfate (Antimicrobiol Agents and Chemotherapy, vol. 44, No. 9, pp. 2492-2497, 2000) and fucoidan (Gastroenteroligy, vol. 119, pp. 358-367, 2000). An example of a substance that inhibits activity of the aforementioned urease is propolis extract. Propolis obtained by means of supercritical extraction is reported to be particularly effective (Medical Nutrition, the issue of January 1, 2003).

Examples of antibiotics include penicillin, cephem, macrolide, neuquinoron, and tetracycline antibiotics. Amoxicillin and clarithromycin antibiotics are particularly preferable. Antibiotic dosages vary depending on the type of agent used. They are generally in the range of 100 mg to 5,000 mg, and preferably in the range of 500 mg to 3,000 mg, per day per adult.

The adhesion inhibitor containing the product of browning reaction of the present invention can be more easily ingested when it is incorporated in foods. The amount of the product of browning reaction of the invention to be incorporated in food is generally about 0.5% to 10% by mass, and preferably 1.0% to 3.0% by mass. Examples of forms of such foods as healthy foods and functional foods include: a variety of pharmaceutical compositions such as fine grains, tablets, granules, capsules, and fluid diet; liquid foods such as soups, juices, tea beverages, milk beverages, cocoa, and jelly-like beverages; semi-solid foods such as puddings and yogurts; bread; snacks; noodles such as *Udon;* snacks such as cookies, chocolates, candies, and rice crackers; and spreads such as Japanese *furikake*, butter and jam. Foods preferably comprise materials containing a large amount of polyphenols, polysaccharides, or glycoproteins capable of eradicating *Helicobacter pylori*. Specific examples thereof include juices, tea beverages, cocoa, or chocolates comprising polyphenol-containing fruit, tea, or cacao, and milk beverages and fermented milk beverages comprising polysaccharides or glycoproteins.

Forms of the products of browning reaction as specified health foods are not particularly limited, although those that can be continuously ingested, such as snacks, dry soups, and beverages are preferable. Also, they can be prepared as diets for patients, such as a low sodium diet, a low caloric diet, or a low protein diet, or as foods for medical use in the form of, for example, soups, beverages, or fluid diet.

Further, foods can contain a variety of food additives, such as a variety of nutrients, vitamins, minerals, dietary fibers, polyunsaturated fatty acids, stabilizing agents such as dispersants or emulsifiers, sweetening agents, taste components, or flavors. Liquid foods may be prepared in such forms. Alternatively, they may be first prepared as powder or paste and then dissolved in a given amount of watery fluid.

PREFERRED EMBODIMENT OF THE INVENTION

The present invention is hereafter described in greater detail with reference to the following examples, although the technical scope of the invention is not limited thereto.

EXAMPLES

Example 1: Browning reaction of casein and lactose in alkaline aqueous solution

Lactose and casein in amounts as shown in the table below were added to 10 ml of a 0.2N NaOH aqueous solution in a 50-ml test tube, and the test tube was shaken with a mixer until a homogenous suspension was formed. The test tube was incubated in a water bath at 50°C for 4 hours to facilitate a browning reaction. 0.2N HCl was added to the solution until pH 7.0. The neutralized solution was lyophilized to give brown powders.

Table 1

| Example | Lactose | Casein |
|---------|---------|--------|
| 1-1 | 100 mg | 900 mg |

Table 1 (continued)

| Example | Lactose | Casein |
|---------|---------|--------|
| 1-2 | 300 mg | 700 mg |
| 1-3 | 400 mg | 600 mg |
| 1-4 | 500 mg | 500 mg |

Example 2: Browning reaction of sodium caseinate and lactose in aqueous solution

Lactose (400 mg) and sodium caseinate (600 mg) were added to 10 ml of distilled water in a 50-ml test tube, and the test tube was shaken with the mixer to dissolve these substances. A browning reaction was carried out in an autoclave at 120°C for 20 minutes. Subsequently, the solution was lyophilized to give brown powders.

Example 3: Browning reaction of β-lactoglobulin and lactose

Lactose (40 mg) and β-lactoglobulin (60 mg) were added to 1.0 ml of a 0.2N NaOH solution in a 15-ml test tube and were dissolved by shaking with the mixer. The test tube was incubated in a water bath at 50°C for 4 hours to facilitate a browning reaction. 0.2N HCl was added to the solution until pH 7.0. The neutralized solution was desalted by gel filtration using the Sephadex G-25 (Amersham Pharmacia) and then lyophilized to give brown powders.

Example 4: Browning reaction of α-lactalbumin and lactose

Lactose (40 mg) and α-lactalbumin (60 mg) were added to 1.0 ml of a 0.2N NaOH solution in a 15-ml test tube and were dissolved by shaking with the mixer. The test tube was incubated in a water bath at 50°C for 4 hours to facilitate a browning reaction. The solution was neutralized and desalted in the same manner as in Example 3 and then lyophilized to give brown powders.

Example 5: Browning reaction of skim milk powder

Milk powder (1.0 g, Kyoupuro E-22, Kyodo Milk Industry Co., Ltd.) was added to 10 ml of a 0.2N NaOH solution. The mixture was stirred until a homogenous suspension was formed. The suspension was incubated in a water bath at 50°C for 4 hours for a browning reaction. The solution was neutralized in the same manner as in Example 3 and then lyophilized to give brown powders.

Example 6: Browning reaction of whey

Whey (1.0 g, Simplesse 100, CP Kelco) was added to 10 ml of a 0.2N NaOH solution. The mixture was stirred until a homogenous suspension was formed. The suspension was incubated in a water bath at 50°C for 4 hours for a browning reaction. The solution was neutralized in the same manner as in Example 3 and then lyophilized to give brown powders.

Example 7: Browning reaction of sodium caseinate and glucose

Glucose (4 g) and sodium caseinate (6 g) were added to 100 ml of distilled water, and the mixture was stirred until solid matters were dissolved. A browning reaction was carried out in an autoclave with the reaction temperature and the reaction period as shown in the table below. After a low-molecular-weight fraction was removed by gel filtration using the Sephadex G-25 (Amersham Pharmacia), the remnant was lyophilized to give brown powders.

Table 2

| Example | Reaction temperature | Reaction period |
|---------|---------------------|-----------------|
| 7-1 | 110°C | 30 minutes |
| 7-2 | 120°C | 10 minutes |
| 7-3 | 120°C | 40 minutes |

Example 8: Browning reaction of sodium caseinate and lactose using sodium bicarbonate

Sodium caseinate (60 mg), lactose (40 mg), and sodium bicarbonate (42 mg) were added to 1 ml of distilled water in a 1.5-ml microtube and were dissolved by stirring. A browning reaction was carried out at 90°C on a heat block for 5 hours. The solution was desalted by gel filtration using the Sephadex G-25 (Amersham Pharmacia) and then lyophilized to give brown powders.

Example 9: Browning reaction of soybean protein

Lactose (400 mg) and soybean protein powder (600 mg, Wako Pure Chemical Industries, Ltd.) were added to 10.0 ml of a 0.2N NaOH solution in a 50-ml test tube and were suspended by shaking with the mixer. The suspension was incubated in a water bath at 40°C for 5 hours to facilitate a browning reaction. 0.6N HCl was added to the solution until pH 7.0. The neutralized solution was lyophilized to give brown powders.

Example 10: Browning reaction of wheat albumin

Lactose (400 mg) and wheat albumin (600 mg, Nisshin Pharma Inc.) were added to 10.0 ml of a 0.2N NaOH solution in a 50-ml test tube and were dissolved by shaking with the mixer. The resultant was incubated in a water bath at 40°C for 5 hours to facilitate a browning reaction. The solution was neutralized in the same manner as in Example 9 and then lyophilized to give brown powders.

Example 11: Browning reaction of egg albumin

Lactose (400 mg) and egg albumin (600 mg, Wako Pure Chemical Industries, Ltd.) were added to 10.0 ml of a 0.2N NaOH solution in a 50-ml test tube and were dissolved by shaking with the mixer. The resultant was incubated in a water bath at 40°C for 5 hours to facilitate a browning reaction. The solution was neutralized in the same manner as in Example 9 and then lyophilized to give brown powders.

Example 12: Browning reaction of zein and lactose

Lactose (400 mg) and zein (600 mg, Wako Pure Chemical Industries, Ltd.) were added to 10.0 ml of a 0.2N NaOH solution in a 50-ml test tube and were dissolved by shaking with the mixer. The solution was incubated in a water bath at 40°C for 5 hours to facilitate a browning reaction. The solution was neutralized in the same manner as in Example 9 and then lyophilized to give brown powders.

Example 13: Browning reaction of casein and xylose using disodium phosphate

Xylose (40 mg) and casein (60 mg) were added to 1.0 ml of an aqueous solution containing 0.1 mol/l disodium hydrogen phosphate in an Eppendorf tube and were suspended by shaking with the mixer. The suspension was incubated in a 85°C heat block for 2 hours to facilitate a browning reaction. After a low-molecular-weight fraction was removed by gel filtration using the Sephadex G-25 (Amersham Pharmacia), the remnant was lyophilized to give brown powders.

Test Example 1: Assay of progress in browning reactions

Progress in browning reactions was evaluated in terms of absorbance at 405 nm.
The products of browning reaction (50 mg each) prepared in Examples 1 to 13 were dissolved in 1 ml of distilled water. The solutions were centrifuged at 10,000xG for 15 minutes, and 100 μl of each supernatant was dispensed per well to a 96-well plate (Nunc Immunoplate; order number: 442404). Absorbance at 405 nm was measured using a microplate reader (Versa Max, Molecular Device) by adopting absorbance at 650 nm as the reference wavelength. As shown in Table 3, all the products of browning reaction prepared in the Examples exhibited absorbance of 0.1 or higher. This indicates that browning reactions made sufficient progress in all Examples.

Table 3

| Examples | Absorbance at 405 nm (reference wavelength: 650 nm) |
|----------|------------------------------------------------------|
| 1-1 | 0.363 |

Table 3 (continued)

| Examples | Absorbance at 405 nm (reference wavelength: 650 nm) |
|---|---|
| 1-2 | 0.956 |
| 1-3 | 1.017 |
| 1-4 | 1.03 |
| 2 | 0.128 |
| 3 | 1.761 |
| 4 | 1.911 |
| 5 | 1.431 |
| 6 | 1.302 |
| 7-1 | 0.164 |
| 7-2 | 0.582 |
| 7-3 | 1.219 |
| 8 | 1.893 |
| 9 | 0.601 |
| 10 | 0.561 |
| 11 | 0.455 |
| 12 | 0.347 |
| 13 | 0.807 |

Test Example 2: Assay of activity of inhibiting *Helicobacter pylori* adhesion

The effects of the products of browning reaction of Examples 1 to 13 for inhibiting the adhesion of a *Helicobacter pylori* adhesin, urease, to the gastric mucosa were evaluated in the following *in vitro* experiments. *Helicobacter pylori* colonizes the host stomach via a specific bond of a *Helicobacter pylori* adhesin, urease, to gastric mucosal mucin. Accordingly, a product that is capable of inhibiting the adhesion between *Helicobacter pylori* urease and gastric mucin with a lower concentration has more potent activity of inhibiting *Helicobacter pylori* adhesion.

(Materials and methods)

Urease and porcine gastric mucin used in the test were prepared in the following manner.

(1) Preparation of urease

A culture solution of *Helicobacter pylori* #130 strains (obtained from Tokai University, School of Medicine, Laboratory of Infectious Disease) in Brucella Broth (3.5 x $10^8$ CFU/ml) was centrifuged at 12,000xG for 20 minutes. The pellets were dissolved in purified water and the solution was mixed using a vortex mixer for 60 seconds. The solution was centrifuged again, and the supernatant of the extract containing urease was obtained. Purification was carried out as described below.

The aforementioned extract was applied to a DEAE-Sephacel column equilibrated with a buffer (20 mM phosphate (pH 6.8), 1 mM EDTA, 1 mM 2-mercaptoethanol, and 10% PEG-300), and then allowed to adsorb to a gel at a flow rate of 0.5 ml/minute. Elution was carried out with a concentration gradient of 0 to 0.5 M KCl. The urease activity of each fraction was monitored. Fractions exhibiting the urease activity peak were pooled and concentrated. Subsequently, the concentrate was applied to a Sephacryl S-300 column equilibrated with a buffer (20 mM phosphate (pH 6.8) and 150 mM NaCl), and then eluted. The urease activity of each fraction was assayed, and the urease activity peaks were pooled, analysed by SDS-PAGE. The result is shown that the fraction of interest comprises a protein of urease A (32 kDa) or urease B (60 kDa). Urease was fractionated and stored at -80°C before use.

(2) Preparation of porcine gastric mucin

A healthy pig (approximately 2 months old) was sacrificed, its stomach was removed, and the inside of the stomach was washed with PBS (pH 7.4) containing 0.1 M phosphate, 0.15 M NaCl, 5 mM N-ethyl maleimide (NEM), 1 mM phenylmethylsulfonyl fluoride (PMSF), and 1 mM EDTA. The stomach was dissected, the mucosa was scraped off, and the scraped mucosa was suspended in the aforementioned buffer. This suspension was homogenized using the Polytron Homogenizer under ice cooling. The homogenate was centrifuged at 15,000xG, and this supernatant was centrifuged again at 25,000xG. The supernatant was recovered, dialyzed with distilled water, and then lyophilized to give roughly purified gastric mucin. Subsequently, the dehydrated and roughly purified gastric mucin was dissolved in PBS (pH 6.8, containing 6 M guanidine hydrochloride and a protease inhibitor (5 mM NEM, 1 mm PMSF, and 1 mM EDTA)), and the solution was layered on cesium chloride density gradient (1.5 g/ml), followed by centrifugation at 34,000xG for 48 hours. Sialic-acid-containing fractions were detected by blotting nitrocellulose membranes and staining using a periodic acid Schiff reagent. Fractions that had developed color were pooled and subjected to the cesium chloride density-gradient centrifugation again. The stained fractions were pooled and lyophilized. Subsequently, gel filtration was carried out using a Sepharose CL-4B column equilibrated with a 0.1 M phosphate buffer (containing 0.1 M NaCl, pH 6,8) and then fractionated. PAS positive fractions with high protein levels were pooled and dialyzed with PBS (pH 6.8), and purified porcine gastric mucin was obtained. The resultant was stored at -80°C before use (purified porcine gastric mucin). The purified porcine gastric mucin was found to consist of 66 kD glycoproteins as a result of SDS-PAGE.

Purified porcine gastric mucin was biotinized and then used in assays in accordance with conventional techniques.

(3) Inhibition test for *Helicobacter pylori* urease adhesion

A 0.05 M sodium carbonate buffer comprising 5 μg/ml *Helicobacter pylori* urease was dispensed per well of a 96-well microplate in amounts of 50 μl each, and the microplate was stored at 4°C overnight to adsorb urease to the wells. Each well was washed three times with 150 μl of 0.05% Tween-20-containing phosphate buffered saline solution (PBS) (pH 7.0), and 150 μl of 3% bovine-serum-albumin-containing PBS (pH 7.0) was then added thereto. The microplate was incubated at 37°C for 1 hour for blocking. After the solution had been removed, the wells were washed three times with 150 μl of 0.05% Tween-20-containing PBS (pH 7.0).

0.05% Tween-20-containing PBS (50 μl, pH 4.0) containing 2.5 μg/ml of biotinized porcine gastric mucin and a given amount of test sample was dispensed to each well, and the microplate was incubated at 37°C for 1 hour to allow the biotinized porcine gastric mucin to adsorb to urease. The solution was removed from the wells, and wells were washed three times with 150 μl of 0.05% Tween-20-containing PBS (pH 4.0). Thereafter, the microplate was incubated at 65°C for 10 minutes to immobilize the proteins thereon. After washing the microplate three times with 150 μl of 0.05% Tween-20-containing PBS (pH 7.0), 50 μl of 0.05% Tween-20-containing PBS (pH 7.0) comprising peroxidase-labeled streptavidin was added thereto, and the resultant was incubated at room temperature for 1 hour. The wells was washed five times with 150 μl of 0.05% Tween-20-containing PBS (pH 7.0), a substrate solution (pH 4.5) containing o-phenylenediamine dihydrochloride and $H_2O_2$ was added to each well in amounts of 50 μl each for a peroxidase reaction. The reaction was proceeded at room temperature for 5 minutes, and 50 μl of 2N sulfuric acid was added to terminate the reaction. Absorbance of each well at 490 nm was assayed.

The activity of inhibiting adhesion was calculated based on the equation below.

Activity of inhibiting adhesion (%) = [1 - (absorbance in a well containing a

sample/absorbance in a well containing no sample)] x 100

(4) Results

The activities of the products of browning reaction of Examples 1 to 13 for inhibiting adhesion at 100 μg/ml were assayed. All the products were found to potently inhibit the adhesion between *Helicobacter pylori* urease and porcine gastric mucin. The results are shown in Table 4.

Table 4:

| Activity of products of browning reaction for inhibiting adhesion | |
| --- | --- |
| Example | Activity of inhibiting adhesion (%) |
| 1-1 | 70 |
| 1-2 | 78 |
| 1-3 | 84 |
| 1-4 | 81 |
| 2 | 60 |
| 3 | 72 |
| 4 | 66 |
| 5 | 82 |
| 6 | 69 |
| 7-1 | 50 |
| 7-2 | 52 |
| 7-3 | 43 |
| 8 | 66 |
| 9 | 63 |
| 10 | 38 |
| 11 | 62 |
| 12 | 16 |
| 13 | 48 |

Test Example 3: *In vivo* experiment of *Helicobacter pylori* eradication

(Method)

NS:Hr/ICR hairless mice (the Institute for Animal Reproduction; Accession No: IAR-NHI-9701, ATCC #72024) (Clin. Diagn. Lab. Immunol. 5, pp. 578-582, 1998) that were highly sensitive to *Helicobacter pylori* infections were employed as experimental animals. Mice were orally inoculated with NSP 335 strains (1 x $10^9$ CFU/mouse) and bred for 1 week. Thereafter, the product of browning reaction of Example 5 or of Example 8 was mixed with feeds, and the feed mixtures were supplied to the mice for 10 weeks. The number of mice in each group was 6. Mice in each group were sacrificed 10 weeks later, their stomachs were removed, the contents therein were removed, and a suspension was then prepared using a homogenizer. The suspension was adopted as a sample for detecting *Helicobacter pylori*.
A medium for detecting *Helicobacter pylori* (Poa Media, a medium for isolating *Helicobacter pylori,* Eiken Chemical Co., Ltd.) was inoculated with the suspension, the resulting medium was cultured by the gas pack method at 37°C for 3 days, and the number of colonies were counted, thereby detecting *Helicobacter pylori*. The group fed with feeds containing no product of browning reaction was determined as a control, and the effect was evaluated by adopting the mean number of colonies of the control group as 100. The results are shown in Table 5 below.

Table 5:

| Effects of eradicating *Helicobacter pylori* | | | |
| --- | --- | --- | --- |
| Browned product | 0.025% feed mixture | 0.25% feed mixture | 2.5% feed mixture |
| Example 5 | 11 | 4 | 12 |
| Example 8 | 48 | 18 | 10 |

As is apparent from the above results, administration of the product of browning reaction of the present invention resulted in a significantly decreased amount of *Helicobacter pylori* in the murine stomach. Also, *Helicobacter pylori* was not detected in some mice in groups administered the products of browning reaction of Example 5 and Example 8. This indicates that the product of browning reaction of the present invention has excellent effects of eradicating *Helicobacter pylori*.

Test Example 4: *In vivo* experiment of *Helicobacter pylori* eradication

The combinatorial effects of the product of browning reaction of the present invention, an inhibitor of gastric-acid secretion, and a variety of substances capable of eradicating *Helicobacter pylori* were tested.

In the same manner as in Test Example 3, *Helicobacter-pylori*-infected mice were fed with one of the products of browning reaction of Example 5 (0.25% feed mixture), Famotidine (0.01% feed mixture), an antibody from chicken egg against *Helicobacter pylori* urease (0.25% feed mixture), milk whey-derived mucin (0.25% feed mixture), sodium caseinate (0.25% feed mixture), catechin (0.25% feed mixture), fucoidan (0.25% feed mixture), omeprazole (0.01 % feed mixture), clarithromycin (0.1% feed mixture), amoxicillin (0.1% feed mixture), dextran sulfate (0.25% feed mixture), propolis extract (0.25% feed mixture), or supercritical fluid extracted propolis (0.25% feed mixture). Alternatively, they were fed with the product of browning reaction in combination with any of the aforementioned substances. *Helicobacter pylori* in the murine stomach was detected 10 weeks later. As a result, single administration of any of the above substances yielded enhanced rate of eradication compared to that obtained by single administration of the product of browning reaction of the invention. The results are shown in Table 6.

Table 6

| Test sample | Effect of eradication |
|---|---|
| Browned product | ++ |
| Famotidine | - |
| Chicken egg antibody | ++ |
| Milk whey-derived mucin | ++ |
| Sodium caseinate | + |
| Catechin | + |
| Fucoidan | + |
| Omeprazole | - |
| Clarithromycin | ++ |
| Amoxicillin | ++ |
| Dextran sulfate | ++ |
| Propolis | ± |
| Supercritical fluid extracted propolis | + |
| Browned product + Famotidine | +++ |
| Browned product + antibody from chicken egg | +++ |
| Browned product + milk whey-derived mucin | +++ |
| Browned product + sodium caseinate | +++ |
| Browned product + catechin | +++ |
| Browned product + fucoidan | +++ |
| Browned product + omeprazole | +++ |
| Browned product + clarithromycin | +++ |
| Browned product + amoxicillin | +++ |
| Browned product + dextran sulfate | +++ |
| Browned product + propolis | +++ |

Table 6   (continued)

| Test sample | Effect of eradication |
|---|---|
| Browned product + supercritically extracted propolis | +++ |

Examples of pharmaceutical compositions comprising the product of browning reaction of the invention and examples of foods comprising the product of browning reaction of the invention are hereafter given.

Example 14: Dry soups

In the manner as described below, dry soups having the following composition were produced.

Meat extract, onion extract, carrot paste, sea tangle extract, salt, and flavor enhancer were mixed and then stirred using a stirrer. An emulsifier was added, and the resultant was stirred. Subsequently, the product of browning reaction and a well-beaten chicken egg were added and then mixed. Finally, spice was added, and mixed with stirring. The resultant was lyophilized to give dry soups.

| | |
|---|---|
| The product of browning reaction of the invention | 23 |
| Chicken egg | 26 |
| Meat extract | 5 |
| Onion extract | 17 |
| Carrot paste | 21 |
| Sea tangle extract | 1 |
| Emulsifier | 1 |
| Salt | 2 |
| Spice (red pepper) | 2 |
| Flavor enhancer (e.g., amino acid) | 2 |
| Total amount | 100 (% by mass) |

Example 15: Fine grains

Fine grains having the following composition were produced by the wet granulation technique.

| | |
|---|---|
| The product of browning reaction of the invention | 45 |
| Lactose (excipient) | 35 |
| Corn starch | 15 |
| Polyvinylpyrrolidone PVP (K-30) | 5 |
| Total amount | 100 (% by mass) |

Example 16: Medical fluid diet

Medical fluid diet (200 ml/package) having the following composition was prepared.

Minerals, sodium phosphate, and potassium phosphate were added to a small amount of water, and the mixture was stirred. The product of browning reaction, maltodextrin, sodium caseinate, an emulsifier, milk protein, and a small amount of water were added thereto, and the resulting mixture was heated to approximately 50°C, followed by stirring. After the aforementioned substances had been suspended, the solution was cooled to room temperature, and vegetable oil, vitamins, spices, a stabilizer, and the rest of water were added to give a medical fluid diet comprising the product of browning reaction.

| | |
|---|---|
| The product of browning reaction of the invention | 3.6 |
| Maltodextrin | 38.0 |
| Sodium caseinate | 13.0 |
| Vegetable oil | 12.0 |
| Vitamins | 1.0 |
| Minerals | 1.5 |

(continued)

| Emulsifier | 0.2 |
| Milk protein | 10.3 |
| Sodium phosphate | 1.8 |
| Potassium phosphate | 1.2 |
| Spice | 0.5 |
| Stabilizer (carragheenan) | 1.5 |
| Water | balance |
| Total amount | 100 (% by mass) |

Example 17: Tablets

Tablets were obtained by a conventional wet compression technique.

| The product of browning reaction of the invention | 40.0 |
| D-mannitol | 20.0 |
| Lactose | 20.0 |
| Crystalline cellulose | 10.0 |
| Hydroxypropyl cellulose | 5.0 |
| Citric acid | 5.0 |
| Total amount | 100 (% by mass) |

Example 18: Tea beverages

In the manner described below, tea beverages (green tea and oolong tea) having the following composition were produced.

A green tea extract was prepared by allowing 3 g of green tea to steep at 85°C for 5 minutes in 100 ml of water. Alternatively, an oolong tea extract was prepared by allowing 4 g of oolong tea to steep at 95°C for 5 minutes in 100 ml of water. The green tea extract or oolong tea extract was mixed with the product of browning reaction at five times the volume thereof, and the mixture was stirred. After the product of browning reaction had become suspended, the rest of tea extract was added to give tea beverages comprising the product of browning reaction.

| The product of browning reaction of the invention | 1 |
| Green tea extract | 99 |
| Total amount | 100 (% by mass) |

Example 19: Cocoa

Cocoa having the following composition was prepared.

Cocoa (Morinaga Milk Industry Co., Ltd.) was added to the product of browning reaction, and the mixture was stirred. Hot water (about 80°C) at five times the volume of the browed product was added thereto, followed by stirring for suspension. After suspension had been achieved, the rest of hot water and milk were added to give cocoa.

| The product of browning reaction of the invention | 5 |
| Cocoa | 5 |
| Milk | 20 |
| Water | 70 |
| Total amount | 100 (% by mass) |

Example 20: Coffee beverages

Coffee beverages having the following composition were prepared.

Coffee grounds (6 g) were extracted at 95°C for 1 minute in 100 ml of water to prepare a coffee extract. The coffee extract at five times the volume of the product of browning reaction and sugar were added, and the mixture was stirred for suspension. The rest of coffee extract and milk were added to give coffee beverages containing the product of browning reaction.

| The product of browning reaction of the invention | 2 |
|---|---|
| Sugar | 1 |
| Milk | 30 |
| Coffee extract | 67 |
| Total amount | 100 (% by mass) |

Example 21: Banana milk shake

A banana milk shake having the following composition was prepared.
The product of browning reaction, milk, and banana were put in a household blender for suspension. Finally, vitamin C was added to give a banana milk shake comprising the product of browning reaction.

| The product of browning reaction of the invention | 2 |
|---|---|
| Vitamin C | 0.5 |
| Milk | 40 |
| Banana | 57.5 |
| Total amount | 100 (% by mass) |

Example 22: *Furikake*

*Furikake* having the following composition was prepared.

| The product of browning reaction of the invention | 40 |
|---|---|
| Flavored dried bonito flakes | 25 |
| Flavored white sesame | 20 |
| Flavored seaweed | 10 |
| Vitamin E | 5 |
| Total amount | 100 (% by mass) |

Example 23: Fermented milk beverages

Lactic bacteria-containing fermented milk (25 g, Yakult) was added to 5 g of the product of browning reaction of the present invention and then was suspended therein. After suspension, 70 g of lactic bacteria-containing fermented milk was added to give fermented milk beverages containing the product of browning reaction.

Example 24: Milk beverages

Milk (25 g) was added to 5 g of the product of browning reaction of the present invention for suspension. After suspension had been achieved, 70 of milk was added to give milk beverage containing the product of browning reaction.

Industrial Applicability

The present invention provides a *Helicobacter pylori* adhesion inhibitor that has excellent activity of eradicating *Helicobacter pylori*, that is highly safe, and that is free from side effects. Unlike conventional antibiotics, the adhesion inhibitor of the present invention can specifically eradicate *Helicobacter pylori* in the stomach without a problem of increased numbers of drug-resistant strains. Accordingly, the *Helicobacter pylori* adhesion inhibitor of the present invention and pharmaceutical compositions and foods comprising the same are useful for preventing and ameliorating diseases associated with *Helicobacter pylori*, such as peptic ulcer.

**Claims**

1.  A *Helicobacter pylori* adhesion inhibitor comprising, as an active ingredient, a product of browning reaction of sugar and protein.

2.  The *Helicobacter pylori* adhesion inhibitor according to claim 1, wherein the protein is at least one member selected from the group consisting of plant proteins derived from wheat, barley, rice, corn, soybeans, or red beans, and animal proteins derived from milk, eggs, fish, or meat.

3.  The *Helicobacter pylori* adhesion inhibitor according to claim 1 or 2, wherein the sugar is at least one member selected from the group consisting of D-glucose, D-fructose, D-mannose, D-galactose, D-xylose, L-arabinose, D-ribose, and lactose.

4.  A method for producing a *Helicobacter pylori* adhesion inhibitor comprising a step of subjecting sugar and protein to a browning reaction in an aqueous solution.

5.  A method for producing a *Helicobacter pylori* adhesion inhibitor comprising a step of subjecting food comprising sugar and protein to a browning reaction in an aqueous solution.

6.  The method according to claim 4 or 5, wherein the protein is at least one member selected from the group consisting of plant proteins derived from wheat, barley, rice, corn, soybeans, or red beans, and animal proteins derived from milk, eggs, fish, or meat.

7.  The method according to claim 4 or 5, wherein the sugar is at least one member selected from the group consisting of D-glucose, D-fructose, D-mannose, D-galactose, D-xylose, L-arabinose, D-ribose, and lactose.

8.  The method according to claim 5, wherein the food is raw cow's milk, milk powder, skim milk powder, whey, or evaporated milk.

9.  The method according to any one of claims 4 to 8, wherein the browning reaction is carried out until absorbance at 405 nm becomes at least 0.01 in a 5% aqueous solution.

10. *A Helicobacter pylori* adhesion inhibitor comprising a product of browning reaction of sugar and protein, and an inhibitor of gastric-acid secretion.

11. A *Helicobacter pylori* adhesion inhibitor comprising a product of browning reaction of sugar and protein, and other substances capable of eradicating *Helicobacter pylori.*

12. The adhesion inhibitor according to claim 11, wherein the substance is at least one member selected from the group consisting of polyphenol, an antibiotic, an antibody against *Helicobacter pylori,* and a polysaccharide or glycoprotein capable of binding to a *Helicobacter pylori* adhesin, urease.

13. A pharmaceutical composition for preventing or treating diseases associated with *Helicobacter pylori* comprising the *Helicobacter pylori* adhesion inhibitor according to any one of claims 1 to 3 and claims 10 to 12.

14. Food for preventing or ameliorating diseases associated with *Helicobacter pylori* comprising the *Helicobacter pylori* adhesion inhibitor according to any one of claims I to 3 and claims 10 to 12.

## INTERNATIONAL SEARCH REPORT

| | International application No. |
|---|---|
| | PCT/JP03/00724 |

**A. CLASSIFICATION OF SUBJECT MATTER**
Int.Cl⁷ A61K38/00, 45/00, 35/20, 31/7004, 31/7016, A61P1/04, 31/04,
A23C9/156, A23F3/00, 5/00, A23L1/22, 1/40, 2/00

According to International Patent Classification (IPC) or to both national classification and IPC

**B. FIELDS SEARCHED**

Minimum documentation searched (classification system followed by classification symbols)
Int.Cl⁷ A61K38/00, 45/00, 35/20, 31/7004, 31/7016, A61P1/04, 31/04,
A23C9/156, A23F3/00, 5/00, A23L1/22, 1/40, 2/00

Documentation searched other than minimum documentation to the extent that such documents are included in the fields searched
Jitsuyo Shinan Koho          1926–1992   Toroku Jitsuyo Shinan Koho   1994–1996
Kokai Jitsuyo Shinan Koho    1971–1992   Jitsuyo Shinan Toroku Koho   1996–2003

Electronic data base consulted during the international search (name of data base and, where practicable, search terms used)
CA(STN), MEDLINE(STN), BIOSIS(STN), EMBASE(STN)

**C. DOCUMENTS CONSIDERED TO BE RELEVANT**

| Category* | Citation of document, with indication, where appropriate, of the relevant passages | Relevant to claim No. |
|---|---|---|
| X | JP 11-12172 A (Yakult Honsha Co., Ltd.),<br>19 January, 1999 (19.01.99),<br>Claims; Par. No. [0011]<br>(Family: none) | 1-14 |
| A | JP 5-238947 A (Yakult Honsha Co., Ltd.),<br>17 September, 1993 (17.09.93),<br>Full text<br>(Family: none) | 1-14 |
| A | JP 10-182451 A (Kabushiki Kaisha Kuree),<br>07 July, 1998 (07.07.98),<br>Full text<br>(Family: none) | 1-14 |

☒ Further documents are listed in the continuation of Box C.          ☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier document but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 23 April, 2003 (23.04.03) | 13 May, 2003 (13.05.03) |

| Name and mailing address of the ISA/ | Authorized officer |
|---|---|
| Japanese Patent Office | |
| Facsimile No. | Telephone No. |

Form PCT/ISA/210 (second sheet) (July 1998)

## INTERNATIONAL SEARCH REPORT

| International application No. |
| --- |
| PCT/JP03/00724 |

**C (Continuation).   DOCUMENTS CONSIDERED TO BE RELEVANT**

| Category* | Citation of document, with indication, where appropriate, of the relevant passages | Relevant to claim No. |
| --- | --- | --- |
| A | JP 10-245344 A  (Ohta's Isan Co., Ltd.),<br>14 September, 1998 (14.09.98),<br>Full text<br>(Family: none) | 1-14 |
| A | WO 98/42323 A1  (TAKEDA CHEMICAL INDUSTRIES, LTD.),<br>01 October, 1998 (01.10.98),<br>Full text<br>& JP 10-324644 A | 1-14 |
| P,A | JP 2002-159566 A  (Maruzen Pharmaceuticals Co., Ltd.),<br>04 June, 2002 (04.06.02),<br>Par. No. [0027]<br>(Family: none) | 1-14 |

Form PCT/ISA/210 (continuation of second sheet) (July 1998)